# EUROPÄISCHE PATENTSCHRIFT

(11) **EP 2 739 259 B1**
(45) Veröffentlichungstag und Bekanntmachung des Hinweises auf die Patenterteilung: **11.10.2017**
(21) Anmeldenummer: 12731458.1
(22) Anmeldetag: 04.07.2012
(51) Int. Cl.: A61K 8/34, A61K 8/41, A61K 8/92, A61Q 19/00, A61Q 5/00

(54) **VERWENDUNG VON SPHINGANIN ZUR VERBESSERUNG DES VISUELLEN ERSCHEINUNGSBILDES VON HAUT UND HAAR**
USE OF SPHINGANINE TO IMPROVE THE VISUAL APPEARANCE OF SKIN AND HAIR
UTILISATION DE SPHINGANINE POUR AMÉLIORER L'ASPECT VISUEL DE LA PEAU ET DES CHEVEUX

(30) Priorität: 03.08.2011 DE 102011109546
(43) Veröffentlichungstag der Anmeldung: 11.06.2014
(73) Patentinhaber: Evonik Degussa GmbH, 45128 Essen (DE)
(72) Erfinder: FARWICK, Mike, 45138 Essen (DE); KÖHLER, Tim, 46284 Dorsten (DE); MENTEL, Matthias, 44357 Dortmund (DE)
(86) Internationale Anmeldenummer: PCT/EP2012/062966
(87) Internationale Veröffentlichungsnummer: WO 2013/017361

(56) Entgegenhaltungen:
- EP-A1- 1 287 815
- WO-A1-94/23694
- WO-A2-02/060406
- CH-A- 399 655
- US-A- 5 882 665
- DATABASE GNPD [Online] MINTEL; Oktober 2010 (2010-10), "Voluminising Detangler", XP002687701, Database accession no. 1418736
- DATABASE GNPD [Online] MINTEL; Februar 2010 (2010-02), "Intensive Repair Masque", XP002687702, Database accession no. 1272283
- DATABASE WPI Week 199747 Thomson Scientific, London, GB; AN 1997-508789 XP002687703, & JP 9 241156 A (NOEVIR KK) 16. September 1997 (1997-09-16)

## Beschreibung

### Gebiet der Erfindung

Gegenstand der Erfindung ist die Verwendung von Sphinganin zur Verbesserung des visuellen Erscheinungsbildes von Haut und Haar.

### Stand der Technik

Ceramide und deren Verwendung in kosmetischen Produkten zur Pflege von Haut und/oder Haar sind seit langem bekannt.

Deren Herstellung ist umständlich.

Aufgabe der Erfindung war es, die Haut- und/oder die Hautanhangsgebilde pflegende Verbindungen bereitzustellen, die ein ähnliches Wirkspektrum wie Ceramide aufweisen, jedoch vereinfacht herzustellen sind.

### Beschreibung der Erfindung

Überraschenderweise wurde gefunden, dass Sphinganin, ein Vorläufer der Ceramide diese Aufgabe zu lösen vermag. Offenbart ist die Verwendung von Sphinganin zur Pflege, von Haut, und Haar. In diesem Zusammenhang sei erwähnt, dass unter Haar auch Fell von Tieren mit eingeschlossen ist.

Sphinganin kann als kosmetischer Wirkstoff zur Pflege der Haut eingesetzt, wobei unterschiedlichste Hauttypen gepflegt werden können, z.B. normale Haut, junge Haut, chronologisch gealterte Haut, photogealterte Haut.

Hierbei wird Sphinganin derart verwendet, dass es die epidermale Lipidbarriere stärkt. Die pflegende Wirkung lässt sich primär mit einer Stärkung der durch Aufnahme und Einlagerung des Sphinganins in entsprechende Barrierelipide erklären.

Somit führt Sphinganin zur Reparatur der geschädigten Lipidbarriere.

Sphinganin besitzt ebenfalls eine protektive Wirkung vor schädlichen intrinsischen sowie extrinsischen Einflussfaktoren.

Die kosmetisch Verwendung von Sphinganin führt generell zu einer Verbesserung der Hautstruktur, insbesondere von gealterter Haut.

Die topische Applikation von Sphinganin auf der Haut führt zu einer Verminderung entzündlicher Reaktionen, wodurch der Hautreizung entgegengewirkt und die Haut beruhigt wird.

Ein weiterer Gegenstand der vorliegenden Erfindung ist Sphinganin zur Behandlung von Akne.

Eine weitere Einsatzmöglichkeit von Sphinganin betrifft die Störung der Hautdesquamationsprozesse. So führt Sphinganin zu einer Normalisierung der natürlichen Keratinozyten-Proliferations- und Desquamationsvorgänge in der Haut, wodurch entsprechenden Störungen

Sphinganin wird vorteilhaft in Kosmetik verwendet, dabei ist die Verwendung von Sphinganin ist nicht auf Leave-on Anwendungen beschränkt. Auch in Form von rinse-off- (z.B. Shampoos, Hautreiniger) und leave-in- (z.B. Haar-Konditionierer) Anwendungen lässt sich Sphinganin vorteilhaft verwenden.

Ein Gegenstand der vorliegenden Erfindung ist Sphinganin zur Reduktion der Fett- bzw. Sebum-Produktion in der Haut, insbesondere der menschlichen Haut.

Dies wiederum führt zu einer Verbesserung des visuellen Erscheinungsbildes der Haut, insbesondere der menschlichen Haut, wodurch Sphinganin erfindungsgemäß kosmetisch verwendet wird, um das visuelle Erscheinungsbild der Haut durch Verringerung des Hautglanzes zu verbessern. Insbesondere auf der Kopfhaut angewandt führt eine erfindungsgemäße, kosmetische Verwendung von Sphinganin zur Verbesserung des visuellen Erscheinungsbildes der Haare, insbesondere menschlicher Haare, durch Verringerung der Sebum Produktion und somit des am Haar haftenden Sebums.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll. Alle angegebenen Prozent (%) sind wenn nicht anders angegeben Massenprozent.

### Folgende Abbildungen sind Bestandteil der Beispiele:

### Abbildung 1:

### Beispiele:

### Beispiel 1: Reduktion der Sebum-Produktion durch Sphinganin

Um die Reduktion der Sebumproduktion der Haut durch topische Applikation von Sphinganin zu demonstrieren, wurde eine humane Sebumstudie durchgeführt.

Das Panel umfasste 30 weibliche Probanden im Alter von 21-45 Jahren, wobei 14 Probanden eine Lotion mit 0,1% Sphinganin bzw. 16 Probanden eine Lotion ohne Sphinganin (Vehikelkontrolle) applizierten. Die Zusammensetzung der Formulierung ist in folgender Tabelle gezeigt:

**Tabelle 1. Testformulierungen. Angaben in Massenprozent.**

| Rohstoff | INCI | Sphinganin-Formulierung | VehikelFormulierung |
|---|---|---|---|
| TEGINACID^{®} C | Ceteareth-25 | 2,0 | 2,0 |
| ABIL^{®} Care 85 | Bis-PEG/PPG-16/16 PEG/PPG16/16 Dimethicone; Caprylic/Capric Triglyceride | 1,0 | 1,0 |
| TEGO^{®} Alkanol 18 | Stearyl Alcohol | 3,5 | 3,5 |
| TEGOSOFT^{®} G20 | Octyl Dodecanol | 5,0 | 5,0 |
| TEGOSOFT^{®} APM | PPG-13 Myristyl Ether | 3,0 | 3,0 |
| TEGOSOFT^{®} DEC | Diethylhexyl Carbonate | 2,0 | 2,0 |
| Sphinganin | Sphinganine | 0,1 | |
| | | | |
| Wasser | Water | 82,68 | 82,78 |
| Milchsäure (10%) | Lactic Acid | 0,5 | 0,5 |
| | | | |
| Euxyl K220 | Methylisothiazolinone, Ethylhexylglycerin, Water | 0,12 | 0,12 |
| Parfüm | | 0,1 | 0,1 |

Zur Herstellung der Formulierungen wurden dem Fachmann bekannte, übliche Formulierungsverfahren eingesetzt.

Die Applikationsphase umfasste 28 Tage, wobei täglich zweimal (morgens und abends) auf der Stirn appliziert wurde.

Gemessen wurde das Hautsebum mittels Sebumeter SM 815^{®} von Courage + Khazaka electronic GmbH, Köln, Deutschland. Die Messungen erfolgten in der Mitte der Stirn im Bereich der Glabellafalte. Registriert wurde der Mittelwert, der sich aus den Einzelwerten von drei Wiederholungsmessungen errechnet. Am Abend vor der jeweiligen Messung wurde keine Formulierung appliziert. Drei Stunden vor der Messung wurde die Stirn mittels eines Feuchttuchs (babylove sensitive Feuchttücher, dm-drogerie markt, Karlsruhe, Deutschland) gereinigt. Die Messungen erfolgten jeweils zu einer für jeden Probanden individuell definierten Tageszeit im Zeitraum zwischen 12:00 und 14:30 Uhr. Bestimmt wurde der Sebumwert zum Zeitpunkt T0 vor der Applikationsphase und T4 nach 28 Tagen.

Wie die humane *in vivo* Studie zeigte, konnte über den Zeitraum von 28 Tagen ein leichter, statistisch aber nicht signifikanter Anstieg der Sebumproduktion in der Gruppe der Personen beobachtet werden, welche die Vehikelformulierung applizierte.

In der Gruppe der Personen, die 0.1 % Sphinganin applizierte, wurde im Vergleich zum Startwert als auch im Vergleich zur Vehikel-Gruppe eine statistisch signifikante Reduktion der Sebumproduktion registriert.

Diese Ergebnisse zeigen, dass aufgrund des Einsatzes von Sphinganin eine signifikante Reduktion der Sebumproduktion erzielt werden kann.

### Beispiel 2: Reduktion der Haarfett-Produktion durch Sphinganin

Um die Reduktion der Haarfett-Produktion durch die Behandlung der Kopfhaut mit Sphinganin zu demonstrieren, wurde eine humane Studie zur Gesundheit der Kopfhaut durchgeführt.

Das Panel umfasste 32 Probanden (gemischt männlich/weiblich, Mindestalter 30, mit Tendenz zu fettiger Kopfhaut /fettigen Haaren), wobei je 16 Probanden ein Haartonikum mit 0,2% Sphinganin bzw. ohne Sphinganin (Vehikelkontrolle) applizierten.

Die Zusammensetzung des Haartonikums ist in folgender Tabelle gezeigt:

**Tabelle 1. Test-Haartonikum. Angaben in Massenprozent**

| Rohstoff | INCI | Sphinganin-Haartonikum | Vehikel-Haartonikum |
|---|---|---|---|
| Teginacid C | Ceteareth-25 | **3** | **3** |
| | | | |
| Sphinganin | Sphinganine | **0,2** | **-** |
| | | | |
| Ethanol | Ethanol | **50** | **50** |
| Wasser | Water | **46,7** | **46,9** |
| Milchsäure (10%) | Lactic Acid | **ad pH 5,5** | **ad pH 5,5** |
| | | | |
| Parfüm | | **0,1** | **0,1** |

Zur Herstellung des Haartonikums wurden dem Fachmann bekannte, übliche Formulierungsverfahren eingesetzt.

Die Applikationsphase umfasste 4 Monate, wobei das Haartonikum täglich zweimal (morgens und abends) mit Hilfe einer Pipette mit einem definierten Volumen von 3 mL auf die Kopfhaut aufgebracht und mit den Händen sanft einmassiert wurde. Die Testparameter umfassten den Glanz des Haares sowie die Fettigkeit der Kopfhaut. Die Fettigkeit der Kopfhaut wird zum einen durch subjektive Bewertung (Selbst- und Experteneinschätzung), zum anderen mittels "'Sebutape"-Methode nach Herstellerangaben durchgeführt.

Zeitpunkte für die Durchführung der Messungen waren vor Beginn der Applikation, nach zwei und nach vier Monaten.

Die Ergebnisse zeigten, dass aufgrund des Einsatzes von Sphinganin eine signifikante Reduktion der Haarfett-Produktion erzielt werden kann.

### Beispiel 3: Beispielformulierungen

**Beispielformulierung 3.1 :Hautstraffende O/W Sonnenschutz-Lotion**

| | |
|---|---|
| Glyceryl Stearate Citrate | 2.00% |
| Cetearyl Alcohol | 1.00% |
| C12-15 Alkyl Benzoate | 5.00% |
| Triisostearin | 1.00% |
| Diethylhexyl Carbonate | 4.00% |
| Octocrylene | 6.00% |
| Bis-Ethylhexyloxyphenol Methoxyphenyl Triazine | 2.00% |
| Ethylhexyl Salicylate | 4.00% |
| Caprylic/Capric Triglyceride; Xymenynic Acid | 1.50% |
| Xanthan Gum | 0.20% |
| Sphinganine | 1.00% |
| Titanium Dioxide; Trimethoxycaprylylsilane; Glycerin | 3.00% |
| Glycerin | 3.00% |
| Water | ad 100.00% |
| Paraffinum Perliquidum | 0.80% |
| Carbomer | 0.20% |
| Sodium Hydroxide (10% in water) | 0.65% |

**Beispielformulierung 3.2 : Leave-in Conditioner**

| | |
|---|---|
| Hydrolyzed Keratin | 2.50% |
| Water | ad 100.00% |
| Sodium Lactate; Sodium PCA; Glycine; Fructose; Urea; Niacinamide; Inositol; Sodium Benzoate; Lactic Acid | 2.00% |
| PEG-40 Hydrogenated Castor Oil | 1.00% |
| Quaternium-80 | 1.50% |
| Ethanol | 15.00% |
| PVP/VA Copolymer | 4.00% |
| Sphinganine | 0.10% |
| Cocamidopropyl Betaine | 4.00% |
| Citric Acid (30%) | 3.00% |

**Beispielformulierung 3.3 : Leave-in Conditioning Spray**

| | |
|---|---|
| Laureth-4 | 0.5% |
| PEG-40 Hydrogenated Castor Oil | 0.5% |
| Sphinganine | 0.1% |
| Quaternium-80 | 0.4% |
| Dimethicone Propyl PG-Betaine | 0.6% |
| Cetrimonium Chloride | 0.8% |
| Water | ad 100.0% |
| Creatine | 0.5% |
| Ethanol | 15.0% |
| PVP/VA Copolymer | 4.0% |
| Sodium Hydroxide (10% in water) | 1.2% |

**Beispielformulierung 3.4: O/W Lotion**

| Formulierung | 3.4.1 | 3.4.2 | 3.4.3 | 3.4.4 | 3.4.5 | 3.4.6 |
|---|---|---|---|---|---|---|
| Decyl Oleate | 5.7% | 5.7% | 5.7% | 5.7% | 5.7% | 5.7% |
| Ethylhexyl Stearate | 7.3% | 7.3% | 7.3% | 7.3% | 7.3% | 7.3% |
| Glyceryl Stearate | 0.5% | 0.5% | 0.5% | 0.5% | 0.5% | 0.5% |
| Stearic Acid | 0.7% | 0.7% | 0.7% | 0.7% | 0.7% | 0.7% |
| Ceteareth-25; Glycerin; Cetyl Alcohol; Behenic Acid; Cholesterol; Ceramide EOP; CeramideEOS; Ceramide NP; Ceramide NS; Ceramide AP; Caprooyl-Phytosphingosine; Caproyl-Sphingosine. | - | 1.0% | - | 0.5% | - | 0.5% |
| Salicyloyl Phytosphingosine | - | - | 0.1% | - | 0.05% | 0.03% |
| Creatine | 0.5% | - | - | 0.2% | 0.1% | 0.2% |
| Cetearyl Glucoside | 1.0% | 1.0% | 1.0% | 1.0% | 1.0% | 1.0% |
| Glycerin | 3.0% | 3.0% | 3.0% | 3.0% | 3.0% | 3.0% |
| Carbomer | 0.2% | 0.2% | 0.2% | 0.2% | 0.2% | 0.2% |
| Sodium Hydroxide (10%) | 0.7% | 0.7% | 0.7% | 0.7% | 0.7% | 0.7% |
| Ethanol | 9.5% | 9.5% | 4.75% | 5.7% | 3.8%% | 7.6% |
| Sphinganine | 0.33% | 0.33% | 0.16% | 0.20% | 0.13% | 0.26% |
| Water | ad 100.0% | ad 100.0% | ad 100.0% | ad 100.0% | ad 100.0% | ad 100.0% |

**Beispielformulierung 3.5: O/W Creme**

| Formulation | 3.5.1 | 3.5.2 | 3.5.3 | 3.5.4 | 3.5.5 | 3.5.6 |
|---|---|---|---|---|---|---|
| Glyceryl Stearate | 2.5% | 2.5% | 2.5% | 2.5% | 2.5% | 2.5% |
| Stearic Acid | 1.0% | 1.0% | 1.0% | 1.0% | 1.0% | 1.0% |
| Stearyl Alcohol | 1.5% | 1.5% | 1.5% | 1.5% | 1.5% | 1.5% |
| Decyl Cocoate | 8.0% | 8.0% | 8.0% | 8.0% | 8.0% | 8.0% |
| Ethylhexyl Stearate | 7.8% | 7.8% | 7.8% | 7.8% | 7.8% | 7.8% |
| Caprylic/Capric Triglyceride | 5.0% | 5.0% | 5.0% | 5.0% | 5.0% | 5.0% |
| Salicyloyl Phytosphingosine | - | - | 0.05% | - | 0.1% | 0.05% |
| Ceramide 3 | - | - | - | 0.05% | - | 0.05% |
| Cetearyl Glucoside | 1.0% | 1.0% | 1.0% | 1.0% | 1.0% | 1.0% |
| Glycerin | 3.0% | 3.0% | 3.0% | 3.0% | 3.0% | 3.0% |
| Carbomer | 0.2% | 0.2% | 0.2% | 0.2% | 0.2% | 0.2% |
| Sodium Hydroxide (10%) | 0.7% | 0.7% | 0.7% | 0.7% | 0.7% | 0.7% |
| Polyglutamic Acid; HydrolisedSclerotium Glucan; Betaine; Urea; Potassium Lactate | 5.0% | 5.0% | 5.0% | 5.0% | 5.0% | 5.0% |
| Hyaluronic Acid | - | 0.05% | - | - | 0.05% | 0.1% |
| Ethanol | 1.9% | 3.8% | 4.75% | 4.75% | 1.9% | 2.85% |
| Sphinganine | 0.07% | 0.13% | 0.16% | 0.16% | 0.07% | 0.10% |
| Water | ad 100.0% | ad 100.0% | ad 100.0% | ad 100.0% | ad 100.0% | ad 100.0% |

**Beispielformulierung 3.6: W/O Lotion**

| Formulation | 3.6.1 | 3.6.2 | 3.6.3 | 3.6.4 | 3.6.5 |
|---|---|---|---|---|---|
| Cetyl PEG/PPG-10/1 Dimethicone | 2.0% | 2.0% | 2.0% | 2.0% | 2.0% |
| Microcristalline Wax | 0.5% | 0.5% | 0.5% | 0.5% | 0.5% |
| Hydrogenated Castor Oil | 0.5% | 0.5% | 0.5% | 0.5% | 0.5% |
| Decyl Oleate | 9.0% | 9.0% | 9.0% | 9.0% | 9.0% |
| Carrylic/Capric Triglyceride | 10.0% | 10.0% | 10.0% | 10.0% | 10.0% |
| Diethylhexyl Carbonate | 5.0% | 5.0% | 5.0% | 5.0% | 5.0% |
| PPG-3 Myristyl Ether; Salicyloyl Phytosphingosine | 3.0% | 3.0% | 3.0% | 3.0% | 3.0% |
| Sodium Chloride | 0.5% | 0.5% | 0.5% | 0.5% | 0.5% |
| Creatine | - | 0.2% | - | | 0.1% |
| Betaine | - | - | 0.3% | | 0.3% |
| Urea | - | - | - | 0.5% | 0.1% |
| Ethanol | 3.8% | 1.9% | 4.75% | 1.9% | 2.85% |
| Sphinganine | 0.13% | 0.07% | 0.16% | 0.07% | 0.1% |
| Water | ad 100.0% | ad 100.0% | ad 100.0% | ad 100.0% | ad 100.0% |

**Beispielformulierung 3.7: Massageöl**

| | |
|---|---|
| Stearylalkohol | 2,0% |
| Petrolatum | 4,0% |
| Dimethicon | 2,0% |
| Isopropylpalmitat | 6,0% |
| Cetylstearylalkohol | 4,0% |
| PEG-40 hydreirtes Rizinusöl | 2,0% |
| Spinganine | 0,2% |
| Glycerin | 3,0% |
| Water | add 100 |

**Beispielformulierung 3.8: Duschgel**

| | |
|---|---|
| PEG-7 Glyceryl Cocoate | 2,0% |
| PEG-40 Hydrogenated Castor Oil | 2,5% |
| Sucrose Cocoate | 2,5% |
| Parfum | 0,5% |
| Water | add 100% |
| Sphinganine | 0,2% |
| Cocamidoprpyl Betaine | 10,5% |
| Sodium Lactate, Sodium PCA Glycine, Fructose, Urea, Niacinamide, Inositol, Sodium benzoate, Lactic Acid, | 1,0% |
| Glycol Distearate, Steateth-4 | 2,0% |

**Beispielformulierung 3.9: Shampoo**

| | |
|---|---|
| Sodium Laureth Sulfate (28%) | 35% |
| Parfum | 0,5% |
| Water | add 100% |
| Sphinganine | 0,2% |
| Quaternium-80 | 0,5% |
| PEG/PPG-4/12 Dimethicone | 0,5% |
| Cocoamidpropyl Betaine | 11,0% |
| PEG-120 Methyl Glucose Dioleate | 0,9% |

**Beispielformulierung 3.10: O/W Deodorant Creme**

| | |
|---|---|
| Zinc Ricinoleate | 2,0% |
| Glyceryl Stearate | 4,0% |
| Isopropyl Myristate | 4,0% |
| Cetyl Alcohol | 2,7% |
| Ceteareth-12 | 2,0% |
| Polyglyceryl-3 Beeswax | 1,0% |
| Sphinganine | 0,1% |
| Glycerin | 3,0% |
| Water | 80,8% |
| Citric acid (50%) | 0,4% |

**Beispielformulierung 3.11: Deo Spray**

| | |
|---|---|
| Zinc Ricinoleate; Triethanolamine; Dipropylene Glycol; Lactic acid | 1,2% |
| Polyglyceryl-3 Caprylate | 0,4% |
| PEG-8 | 1,4% |
| Cyclomethicone | 1,4% |
| Alcohol denat. | 25,6% |
| Sphinganine | 0,1% |
| Propane/Butane | 69,9% |

**Beispielformulierung 3.12: 24h AP/Deo Stick**

| | |
|---|---|
| PPG-11 Stearyl Ether | 5,0% |
| PPG-3 Myristyl Ether | 5,0% |
| Stearyl Alcohol | 16,25% |
| Hydrogenated Castor Oil | 1,75% |
| Sphinganine | 0,2% |
| Cyclopentasiloxane (and) Cyclohexasiloxane | 44,3% |
| Silica Dimethylsilylate | 3,0% |
| Aluminium Chlorohydrate | 20,0% |
| Polyglyceryl-3 Caprylate | 0,5% |
| Zinc Ricinoleate; Lysine; Propylene | 4,0% |

**Beispielformulierung 3.13: Deo Roll-On**

| | |
|---|---|
| Polyglyceryl-3 Caprylate | 0,5% |
| Laureth-23 | 3,0% |
| Sphinganine | 0,1% |
| Parfum | 0,5% |
| PEG-14 Dimethicone | 0,5% |
| Alcolhol | 20% |
| PEG-7 Glyceryl Cocoate | 1,0% |
| Water | 16,7% |
| Allantoin | 0,2% |
| Panthenol | 0,1% |
| Aluminium Chlorohydrate, 50% Solution | 20,0% |
| Hydroxyethylcellulose | 0,75% |
| Water | 36,65% |
| Preservative | q.s. |

**Beispielformulierung 3.14: Reinigungs-Tonikum**

| | |
|---|---|
| Polyglyceryl-4 Caprate | 1,0% |
| Phytosphingosine Hydrochloride | 0,05% |
| Sphinganine | 0,05% |
| Ethanol | 10,0% |
| Perfume | 0,2% |
| Water | 87,6% |
| Sodium Lactate; Sodium PCA; Glycine; Fructose; Urea; niacinamide; Inositol; Sodium Benzoate; Lactic Acid | 1,0% |
| Panthenol | 0,1% |
| Preservative | q.s. |

**Beispielformulierung 3.15: Reinigender Gesichtsschaum**

| | |
|---|---|
| Disodium PEG-5 Laurylcitrate Sulfosuccinate; Sodium Laureth Sulfate | 8,0% |
| Sodium Cocoamphoacetate | 12,0% |
| Capryl/Capramidopropyl Betaine | 2,0% |
| Polyglyceryl-3 Caprate | 0,3% |
| PPG-3 Myristyl Ether | 0,5% |
| Sphinganine | 0,1% |
| Water | 77,2% |
| D-Panthenol | 0,2% |
| Creatine | 0,5% |
| Methoxy PEG/PPG-7/3 Aminopropyl Dimethicone | 0,2% |

**Beispielformulierung 3.16: Konditionierendes Anti-Schuppen Shampoo**

| | |
|---|---|
| Glycol Distearate | 3,0% |
| Sodium Laureth Sulfate, 28% | 40,0% |
| Sphinganine | 0,05% |
| Perfume | 0,3% |
| Zinc Pyrithione, 48% | 2,0% |
| Quaternium-80 | 1,0% |
| Water | 36,65% |
| Acrylates/C10-30 Alkyl Acrylate Crosspolymer | 0,2% |
| Polyquaternium-10 | 0,3% |
| NaOH, 25% | 0,3% |
| Undecylenamidopropyl Betain | 12,5% |
| Isostearamide MIPA | 3,5% |

**Beispielformulierung 3.17: Shampoo**

| | |
|---|---|
| Sodium Lauryl Sulfate, 28% | 50,0% |
| Sucrose Cocoate | 2,9% |
| Perfume | 0,5% |
| Quaternium-80 | 0,3% |
| Water | 34,45% |
| D-Panthenol | 1,5% |
| Cocamidopropyl Betaine | 6,4% |
| PEG-18 Glyceryl Oleate/Cocoate | 1,0% |
| Glycol Distearate; Steareth-4 | 2,5% |
| Sphinganine | 0,05% |
| Preservative | q.s. |
| NaCl | q.s. |

**Beispielformulierung 3.18: Konditionierende Haarspülung**

| | |
|---|---|
| Ceteareth-25 | 0,5% |
| Cetearyl Alcohol | 2,5% |
| Distearyldimonium Chloride | 1,0% |
| Isocetyl Alcohol; Ceramide NP; Cetyl Alcohol | 2,0% |
| Glyceryl Stearate | 1,5% |
| Sphinganine | 0,05% |
| Glycerin | 3,0% |
| Quaternium-80 | 1,0% |
| Water | 88,45% |
| Citric acid | ad pH=4 |
| Preservative | q.s. |
| Perfume | q.s. |

**Beispielformulierung 3.19: Zahncreme**

| | |
|---|---|
| Dicalcium Phosphate | 47,5% |
| Glycerin, 86% | 30,0% |
| Flavor | 1,0% |
| Saccharin, 1% | 0,5% |
| Sodium Lauryl Sulfate | 1,0% |
| Sphinganine | 0,1% |
| Water | 19,9% |

**Beispielformulierung 3.20: Anti-Karies Gel**

| | |
|---|---|
| Glycerin | 10,0% |
| Sodium Metaphosphate | 30,0% |
| Titanium Dioxide | 1,0% |
| Silica | 3,0% |
| Cetylamine Hydrofluoride | 1,0% |
| Olaflur | 2,0% |
| Methylparaben | 0,15% |
| Mineral oil | 1,0% |
| Saccharin | 0,03% |
| Menthol | 0,2% |
| Flavor/Aroma | 1,0% |
| Cocamidopropyl Betaine | 3,0% |
| Sphinganine | 0,1% |
| Water | 47,52% |

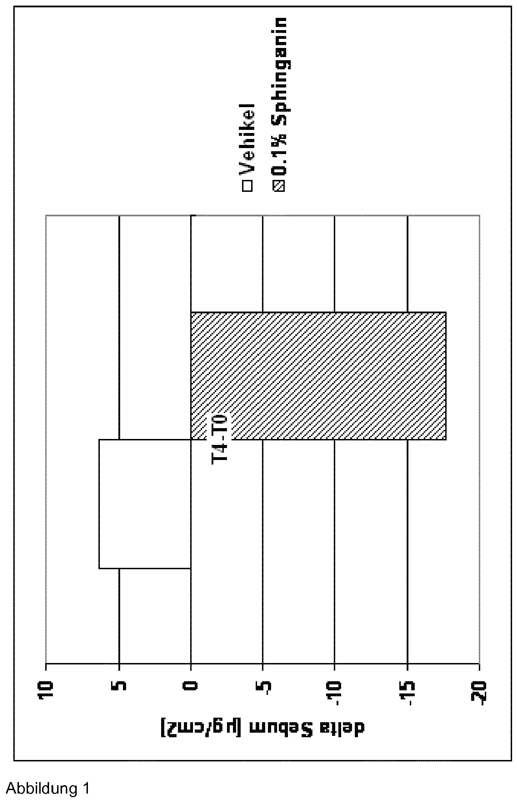

## Patentansprüche

1. Sphinganin zur Verwendung in der Behandlung von Akne.

2. Kosmetische Verwendung von Sphinganin zur Verbesserung des visuellen Erscheinungsbildes der Haare oder der Haut durch Verringerung der Sebumproduktion.

## Claims

1. Sphinganine for use in the treatment of acne.

2. Cosmetic use of sphinganine for improving the visual appearance of the hair or of the skin by reducing sebum production.

## Revendications

1. Sphinganine destinée à une utilisation dans le traitement de l'acné.

2. Utilisation cosmétique de sphinganine pour améliorer l'aspect visuel des cheveux ou de la peau par réduction de la production de sébum.
